# EUROPEAN PATENT APPLICATION

(11) **EP 2 955 184 A1**
(43) Date of publication of application: **16.12.2015**
(21) Application number: 14749294.6
(22) Date of filing: 10.01.2014
(51) Int. Cl.: C07D 491/22, C07D 491/147

(54) **METHOD FOR PREPARING (+)-TRICYCLIC HYDROXYL LACTONE**

(30) Priority: 05.02.2013 CN 201310045202
(71) Applicant: Fudan University, Shanghai 200433 (CN)
(72) Inventor: CHEN, Fener, Shanghai 200433 (CN); HE, Qiuqin, Shanghai 200433 (CN); XIONG, Fangjun, Shanghai 200433 (CN); CHEN, Wenxue, Shanghai 200433 (CN); WANG, Xinlong, Shanghai 200433 (CN)
(74) Representative: Kånge, Lars Rikard
(86) International application number: PCT/CN2014/070438
(87) International publication number: WO 2014/121671

(57) **Abstract**

The invention belongs to the technical field of organic chemistry, in particular being a method for preparing (+)-tricyclic hydroxyl lactone. The preparation of the (+)-tricyclic hydroxyl lactone compound in the prior art has lengthy steps, low stereoselectivity and high costs. The (+)-tricyclic hydroxyl lactone of the invention is obtained by an asymmetric oxidation reaction of prochiral tricyclic lactones in an organic solvent with an optically active Davis oxidant in the presence of an organic base. The method of the invention uses easily available raw materials, has low costs, good selectivity, and is suitable for large-scale preparation.

## Description

### Field of the Invention

The invention belongs to the technical field of organic chemistry, and in particular relates to a method for preparing (+)-tricyclic hydroxyl lactone (I).

### Background of the Invention

(+)-tricyclic hydroxyl lactone is the key chiral intermediate for the asymmetric synthesis of anti-tumor medicines such as camptothecin, 10-hydroxycamptothecin, topotecan and irinotecan and the like. Its structure is shown in formula (I): 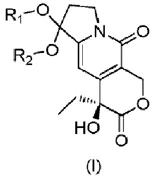 wherein R₁ and R₂ are connected or unconnected branched or straight C1-C5 alkyl.

Wall et al. (J. Med. Chem. 1980, 23, 554; J. Med. Chem. 1986, 29, 1553; J. Med. Chem. 1986, 29, 2358; J. Med. Chem. 1987, 30, 1774) disclosed that a racemic tricyclic hydroxyl lactone could be produced by oxidizing tricyclic lactone via introducing oxygen under basic condition and subsequently resolved and cyclized by using *S*-phenethylamine as the chiral adjuvant to produce (+)-tricyclic hydroxyl lactone. The disadvantages of this method include long reaction path, using stoichiometric *S*-phenethylamine as the chiral adjuvant and low yield. Tagawa et al. (Chem. Pharm. Bull. 1989, 37, 3382; Tetrahedron Lett. 1989, 30, 2639; J. Chem. Soc., Perkin Trans. 1 1990, 27; US Patent No. 4778891) firstly mesylated (-)-tricyclic hydroxyl lactone; then the configuration was inversed by conducting cesium acetate SN2 nucleophilic substitution and (+)-tricyclic hydroxyl lactone was produced through hydrolysis. However, this method uses cesium acetate which is expensive and is not industrially accessible as an nucleophilic reagent. Imura et al. (Tetrahedron: Asymmetry 1988, 9, 2285) reported using papain to resolve racemic tricyclic hydroxyl lactone to produce (+)-tricyclic hydroxyl lactone. However, the enzyme is not industrially accessible and not suitable for large-scale preparation. Furthermore, Jew et al. (Tetrahedron: Asymmetry 1995, 6, 1245) developed a method of preparing (+)-tricyclic hydroxyl lactone, which uses racemic tricyclic lactone as a raw material and comprises five steps including reduction, mesylation, dehydration and Sharpless asymmetric dihydroxylation reaction. However, this method is of low stereoselectivity and uses the Sharpless reagent which is difficult to recycle.

### Summary of the Invention

The object of the invention is to overcome the disadvantages of the prior art and provide a method of preparing (+)-tricyclic hydroxyl lactone with convenience, low costs and high stereoselectivity.

The invention provides a method of preparing (+)-tricyclic hydroxyl lactone (I), which comprises using an optically active Davis oxidant to asymmetrically oxidize prochiral tricyclic lactone (II) in an organic solvent in the presence of an organic strong base to produce optically active (+)-tricyclic hydroxyl lactone. The total yield is more than 70% and ee is more than 75%. The synthetic scheme is shown as follows: 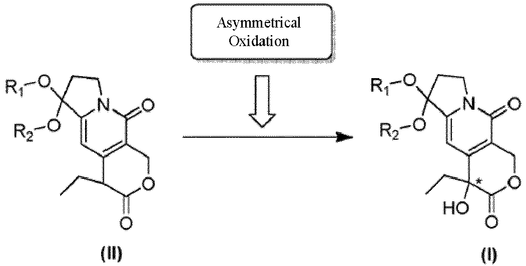 wherein R₁ and R₂ are connected or unconnected branched or straight C1-C5 alkyl.

The organic base used in the invention is alkali metal hexamethyldisilazide such as any of sodium hexamethyldisilazide, potassium hexamethyldisilazide and lithium hexamethyldisilazide or alkali metal amide such as any of potassamide, sodamide and lithamide or C1-C4 lithium alkylide such as any of n-butyllithium and t-butyllithium, all of which can facilitate the reaction.

The chiral Davis oxidant described in the invention is any of (+)-camphorsulfonyl azaoxacyclopropane, all of which possess the effect of highly enantioselective oxidization. Such chiral Davis oxidant can be prepared according to the reference (Tetrahedron Lett. 1989, 30, 1613; J. Org. Chem. 1997, 62, 6093**,** etc.). The structure of (+)-camphorsulfonyl azaoxacyclopropane is shown as follows: 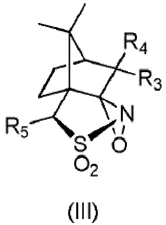 wherein R₃ and R₄ are identical and are hydrogen, methoxyl or chlorine; R₅ is p-trifluoromethyl benzyl or PS-CH₂.

The molar ratio of the prochiral tricyclic lactone (II) / the organic base / the Davis oxidant in the invention is 1 : 0.8-3 : 1-3.

The organic solvent used in the invention is any of dichloromethane, dichloroethane, tetrahydrofuran, dioxane and *N,N*-dimethylformamide as well as a mixture of several of them in any proportion. These solvents are easily available and cheap.

The suitable temperature of the invention is -78 to -40 °C and the reaction time is 5-20 h.

The advantageous reaction conditions of the invention are that:
the organic base is potassium hexamethyldisilazide, with the best yield;
the optically active Davis oxidant is (+)-camphorsulfonyl azaoxacyclopropane (III) (R₃=R₄=methoxyl and R₅=p-trifluoromethyl benzyl), with the best stereoselectivity;
the molar ratio of compound (II) / the organic base / the Davis oxidant is 1 : 1.1-2.5 : 1.5-2, with a good yield;
the organic solvent is tetrahydrofuran or dichloromethane, with a good yield;
the reaction time is 5-10 h and the reaction temperature is -78 to -55 °C, with a good yield and stereoselectivity.

The method of the invention uses easily available raw materials, has low costs, good selectivity, and is suitable for large-scale preparation.

### Detailed Description of the Invention

The invention is further described according to particular examples as follows. However, the scope of the invention is not limited to that.

### I. The preparation of (5'S)-1,1-(2,2-dimethyl-1,3-propylenedioxy)-5-oxo-(5'-ethyl-5'-hydroxyl-2'H,5'H,6'H-6-oxopyran)-[3',4',f}-Δ6(8)-tetrahydroindolizine ((+)-Tricyclic Hydroxyl Lactone, I)

### Example 1

Under the protection of nitrogen, compound 1,1-(2,2-dimethyl-1,3-propylenedioxy)-5-oxo-(5'-ethyl-2'H,5'H,6'H-6-oxopyran)-[3',4',f]-Δ⁶⁽⁸⁾-tetrahydroindolizine (tricyclic lactone, II) (3.33 g, 0.01 mol) was dissolved in anhydrous tetrahydrofuran (10 ml) and placed in a dry flask. The mixture was stirred for 30-45 min at -60 °C and a solution of potassium hexamethyldisilazide in tetrahydrofuran (0.85 M, 12.94 ml) and DMF (5 ml) were added dropwise subsequently and slowly. The mixture was stirred for 1 hour and then a solution of (+)-camphorsulfonyl azaoxacyclopropane (III) (R₃=R₄=methoxyl and R₅=p-trifluoromethyl benzyl) (6.88 g, 0.0154 mmol) in tetrahydrofuran (10 ml) was added dropwise. After reacting for 8 hours at -60 °C, the reaction was complete. The reactants were poured into water and extracted with dichloromethane and the organic layers were combined. After washing with water and drying as well as recycling the solvents under reduced pressure, the reactants were cooled to room temperature and recrystallized by using absolute ethanol to produce 2.55g (5'S)-1,1-(2,2-dimethyl-1,3-propylenedioxy)-5-oxo-(5'-ethyl-5'-hydroxyl-2'H,5'H,6'H-6-oxopyran)-[3',4',f]-Δ6(8)-tetrahydroindolizine as white crystalline powder, mp209-210 °C, [α]_{D}²⁵=+89.5 (c0.99,CHCl₃), ee=96%, yield 73%.
**¹H NMR** (CDCl₃): δ=6.81 (s, 1H), 5.62 *(d,* 1H, *J*=16 Hz), 5.19 *(d,* 1H, *J*=16 Hz), 4.12-4.17 *(m,* 2H), 3.74 (*s*, 1H), 3.64-3.71 *(m,* 4H), 2.54 (*t,* 2H, *J*=6.8 Hz), 1.77-1.86 (*m,* 2H), 1.29 (s, 3H), 0.99 (*t,* 3H, *J*=7.2 Hz), 0.87 (s, 3H).
**ESI-MS:** (m/z)=350 (M+H, base peak)

### Example 2

Under the protection of nitrogen, compound 1,1-(2,2-dimethyl-1,3-propylenedioxy)-5-oxo-(5'-ethyl-2'H,5'H,6'H-6-oxopyran)-[3',4',f]-Δ⁶⁽⁸⁾-tetrahydroindolizine (tricyclic lactone, II) (3.33 g, 0.01 mol) was dissolved in anhydrous tetrahydrofuran (10 ml) and placed in a dry flask. The mixture was stirred for 30-45 min at -60 °C and a solution of sodium hexamethyldisilazide in tetrahydrofuran (0.85 M, 17.65 ml) and DMF (5 ml) were added dropwise subsequently and slowly. The mixture was stirred for 1 hour and then a solution of (+)-camphorsulfonyl azaoxacyclopropane (III) (R₃=R₄=methoxyl and R₅=p-trifluoromethyl benzyl) (6.88 g, 0.0154 mmol) in tetrahydrofuran (10 ml) was added dropwise. After reacting for 10 hours at -60 °C, the reaction was complete. The reactants were poured into water and extracted with dichloromethane and the organic layers were combined. After washing with water and drying as well as recycling the solvents under reduced pressure, the reactants were cooled to room temperature and recrystallized by using absolute ethanol to produce 2.44g (5'S)-1,1-(2,2-dimethyl-1,3-propylenedioxy)-5-oxo-(5'-ethyl-5'-hydroxyl-2'H,5'H,6'H-6-oxopyran)-[3',4',f]-Δ6(8)-tetrahydroindolizine as white crystalline powder, mp208-210 °C, ee=87%, yield 70%. The ¹H NMR and ESI-MS data is identical to that of Example 1.

### Example 3

Under the protection of nitrogen, compound 1,1-(2,2-dimethyl-1,3-propylenedioxy)-5-oxo-(5'-ethyl-2'H,5'H,6'H-6-oxopyran)-[3',4',f]-Δ6⁽⁸⁾-tetrahydroindolizine (tricyclic lactone, II) (3.33 g, 0.01 mol) was dissolved in anhydrous tetrahydrofuran (10 ml) and placed in a dry flask. The mixture was stirred for 30-45 min at -60 °C and a solution of lithium hexamethyldisilazide in tetrahydrofuran (0.85 M, 23.53 ml) and DMF (5 ml) were added dropwise subsequently and slowly. The mixture was stirred for 1 hour and then a solution of (-)-camphorsulfonyl azaoxacyclopropane (III) (R₃=R₄=hydrogen and R₅=p-trifluoromethyl benzyl) (7.74 g, 0.02 mmol) in tetrahydrofuran (10 ml) was added dropwise. After reacting for 10 hours at -60 °C, the reaction was complete. The reactants were poured into water and extracted with dichloromethane and the organic layers were combined. After washing with water and drying as well as recycling the solvents under reduced pressure, the reactants were cooled to room temperature and recrystallized by using absolute ethanol to produce 2.48g (5 'S)-1,1-(2,2-dimethyl-1,3-propylenedioxy)-5-oxo-(5'-ethyl-5'-hydroxyl-2'H,5'H,6'H-6-oxopyran)-[3',4',f]-Δ6(8)-tetrahydroindolizine as white crystalline powder, mp208-210 °C, ee=76%, yield 71%. The ¹H NMR and ESI-MS data is identical to that of Example 1.

### II. The preparation of (5'S)-1,1-ethylenedioxy-5-oxo-(5'-ethyl-5'-hydroxyl-2'H,5'H,6'H-6-oxopyran)-[3',4',f]-Δ6(8)-tetrahydroindolizine ((+)-Tricyclic Hydroxyl Lactone, I)

### Example 4

Under the protection of nitrogen, compound 1,1-ethylenedioxy-5-oxo-(5'-ethyl-2'H,5'H,6'H-6-oxopyran)-[3',4',f]-Δ⁶⁽⁸⁾-tetrahydroindolizine (tricyclic lactone, II) (0.29 g, 1mmol) was dissolved in anhydrous tetrahydrofuran (30 ml) and placed in a dry flask. The mixture was stirred for 30-45 min at -60 °C and a solution of potassium hexamethyldisilazide in tetrahydrofuran (0.85 M, 1.29 ml) was added dropwise subsequently and slowly. The mixture was stirred for 1 hour and then a solution of (+)-camphorsulfonyl azaoxacyclopropane (III) (R₃=R₄=methoxyl and R₅=polyethylene benzyl) (15 g, 1.2 mmol) was added in batches. After reacting for 10 hours at -60 °C, the reaction was complete. The reactants were poured into water and filtered and the water layer was extracted with dichloromethane and the organic layers were combined. After washing with water and drying as well as recycling the solvents under reduced pressure, the reactants were cooled to room temperature and recrystallized by using absolute ethanol to produce 0.27g (5'S)-1,1-ethylidenedioxy-5-oxo-(5'-ethyl-5'-hydroxyl-2'H,5'H,6'H-6-oxopyran)-[3',4',f]-Δ6(8)-tetrahydroindolizine as white crystalline powder, mp170-172 °C, [α]_{D}²⁵=+108.8 (c0.76,CHCl₃), ee=94%, yield 88%.
**¹H NMR** (CDCl₃): δ=6.58 (s, 1H), 5.62 *(d,* 1H, *J*=16 Hz), 5.18 *(d,* 1H, *J*=16 Hz), 4.13-4.21 *(m,* 6H), 3.76 (*s*, 1H), 2.43 (*t,* 2H, *J*=6.8 Hz), 1.78-1.82 (*m,* 2H), 0.98 (*t,* 3H, *J*=7.2 Hz).
**ESI-MS:** (m/z)=308 (M+H, base peak)

All the references in the invention are incorporated herein by reference in its entirety as if each individual reference was specifically and individually indicated to be incorporated by reference. It is apparent to those skilled in the art in light of the teachings of this invention that certain changes and modifications may be made thereto. Such equivalent forms also fall into the scope of the appended claims.

## Claims

1. A method for preparing (+)-tricyclic hydroxyl lactone having the structure of formula (I): 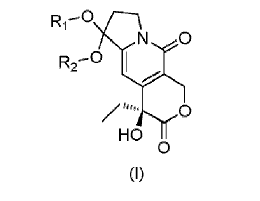 wherein R₁ and R₂ are connected or unconnected branched or straight C1-C5 alkyl;
wherein the method comprises: using an optically active Davis oxidant to asymmetrically oxidize prochiral tricyclic lactone (II) in an organic solvent in the presence of an organic base to produce optically active (+)-tricyclic hydroxyl lactone of formula (I), the synthetic scheme of which is shown as follows: 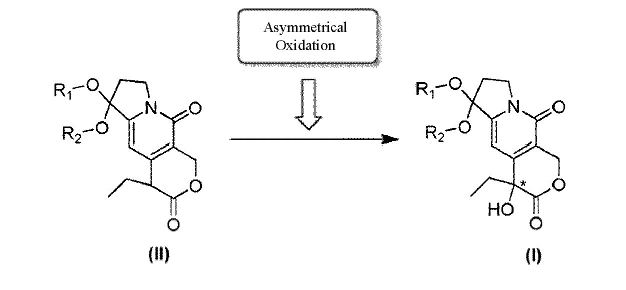 wherein the conditions for preparation are that:
(1) the organic base used is alkali metal hexamethyldisilazide or alkali metal amide or C1-C4 lithium alkylide;
(2) the Davis oxidant used is (+)-camphorsulfonyl azaoxacyclopropane, the structure of which is shown by formula (III): 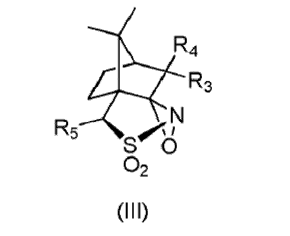 wherein R₃ and R₄ are identical and are hydrogen, methoxyl or chlorine; R₅ is p-trifluoromethyl benzyl or PS-CH₂;
(3) the molar ratio of the prochiral tricyclic lactone (II) / the organic base / the Davis oxidant is 1 : 0.8-3 : 1-3;
(4) the organic solvent used is any one of dichloromethane or dichloroethane, tetrahydrofuran, dioxane and *N,N-*dimethylformamide as well as a mixture of several of them;
(5) the reaction temperature is -78 to -40 °C;
(6) the reaction time is 5-20 h.

2. The method of claim 1, wherein the organic base is potassium hexamethyldisilazide.

3. The method of claim 1, wherein the optically active Davis oxidant is (+)-camphorsulfonyl azaoxacyclopropane (III), and wherein R₃=R₄=methoxyl and R₅=p-trifluoromethyl benzyl.

4. The method of claim 1, wherein the molar ratio of compound (II) / the organic base / the Davis oxidant is 1 : 1.1-2.5 : 1-2.

5. The method of claim 1, wherein the reaction temperature is -78 to -55 °C and the reaction time is 5-10 h.
